# FASCICULE DE BREVET EUROPEEN

(11) **EP 0 804 574 B1**
(45) Date de publication et mention de la délivrance du brevet: **31.01.2007**
(21) Numéro de dépôt: 95925024.2
(22) Date de dépôt: 07.07.1995
(51) Int. Cl.: C12N 15/12, C07K 14/705, C12Q 1/68, G01N 33/68, A61K 38/17

(54) **RECEPTEUR OPIOIDE KAPPA HUMAIN, ACIDES NUCLEIQUES ET UTILISATIONS**
HUMANER KAPPA OPIATREZEPTOR, NUKLEINSÄUREN UND IHRE VERWENDUNGEN
HUMAN KAPPA OPIOID RECEPTOR, NUCLEIC ACIDS AND USES THEREOF

(30) Priorité: 11.07.1994 FR 9408531
(43) Date de publication de la demande: 05.11.1997
(73) Titulaire: AstraZeneca AB, 151 85 Södertälje (SE)
(72) Inventeur: KIEFFER, Brigitte, 67150 Erstein-Krafft (FR); SIMONIN, Frédéric, 67300 Schiltigheim (FR)
(74) Mandataire: Almond-Martin, Carol
(86) Numéro de dépôt international: PCT/FR1995/000912
(87) Numéro de publication internationale: WO 1996/001898

(56) Documents cités:
- WO-A-94/28132
- FEBS LETTERS, vol. 202, no. 3, 15 Août 1994 AMSTERDAM NL, pages 1431-1437, MANSSON E., BARE L., YANG D. 'Isolation of a human kappa opioid receptor cDNA from placenta'
- PROCEEDINGS OF THE NATIONAL ACADEMY OF SCIENCES OF USA, vol. 91, Avril 1994 WASHINGTON US, pages 3779-3783, XIE G.X., MENG F., MANSOUR A., THOMPSON R.C., HOVERSTEN M.T.,GOLDSTEIN A., WATSON S.J., AKIL H.; 'Primary structure and functional expression of a guinea pig kappa pioid (dynorphin) receptor'
- PROCEEDINGS OF THE NATIONAL ACADEMY OF SCIENCES OF USA., vol. 90, Novembre 1993 WASHINGTON US, pages 9954-9958, MENG, F. ET AL.; 'Cloning and pharmacological characterization of a rat k opioid receptor'
- FEBS LETTERS., vol. 330, no. 1, Septembre 1993 AMSTERDAM NL, pages 77-80, NISHI, M. ET AL.; 'cDNA cloning and pharmacological characterization of an opioid receptor with high affinities for kappa-subtype -selective ligands'
- FEBS LETTERS., vol. 329, no. 3, Août 1993 AMSTERDAM NL, pages 291-295, MINAMI, M. ET AL.; 'Cloning and expression of a cDNA for the rat kappa-opioid receptor'
- PROCEEDINGS OF THE NATIONAL ACADEMY OF SCIENCES OF USA., vol. 90, Juillet 1993 WASHINGTON US, pages 6736-6740, YASUDA, K. ET AL.; 'Cloning and functional comparison of kappa and delta opioid receptors from mous brain'

## Description

La présente invention concerne de nouveaux polypeptides et le matériel génétique permettant leur expression Plus particulièrement, elle concerne de nouveaux polypeptides ayant une activité de récepteur opioïde kappa humain.

Les récepteurs opioïdes sont des récepteurs membranaires du système nerveux qui médient les propriétés analgésiques et psychotropes des drogues alkaloïdes de type morphinique (Brownstein, 1993, Proc. Natl. Acad. Sci. USA, vol 90, 5391). Les études pharmacologiques ont démontré l'existence de trois sous-types de récepteurs, nommés mu, delta et kappa, qui différent par leur capacité à lier les différents ligands opioïdes (Goldstein et al, 1989, Mol. Pharmacol., vol 36, 265-272.) et par leur distribution dans l'organisme (Mansour et al, 1987, J. Neurosci., vol 7, 2445). Récemment, trois récepteurs opioïdes ont été clonés chez les rongeurs. Le profil pharmacologique de ces trois récepteurs clonés exprimés transitoirement en cellules Cos indiquent qu'il s'agit d'un récepteur delta, d'un récepteur mu et d'un récepteur kappa. L'analyse de leur structure primaire confirme qu'ils font partie de la famille des récepteurs couplés aux protéines G, qui possèdent une topologie putative à sept domaines transmembranaires (Bockaert, 1991, Curr. Op. Neurobiol., vol 1, 32).

La présente invention réside dans la mise en évidence, l'isolement et la caractérisation moléculaire du récepteur opioïde kappa humain. Elle réside notamment dans l'isolement et le séquençage du gène codant pour ce récepteur, dans la construction de souches recombinées permettant l'expression de récepteurs fonctionnels, et dans la mise au point de tests permettant l'isolement de composés actifs sur ces récepteurs et possédant des propriétés thérapeutiques avantageuses. Les séquences d'ADN de l'invention permettent également la réalisation de sondes, capables de détecter dans des échantillons biologiques toute irrégularité dans l'expression d'un récepteur opioïde kappa (non-expression, mutation, polymorphisme, etc). Ces sondes sont également utilisables pour le clonage par hybridation de tout autre ADNc codant pour un récepteur opioïde, à partir de tissus d'origines diverses et notamment d'origine humaine.

Un premier objet de l'invention réside donc dans une séquence nucléotidique codant pour un polypeptide ayant une activité de récepteur opioïde kappa humain.

Plus préférentiellement, la séquence nucléotidique selon l'invention est choisie parmi les séquences codant pour un polypeptide ayant une activité de récepteur opioïde kappa humain, ledit polypeptide comprenant
a. la séquence peptidique SEQ ID n°1, ou
b. un dérivé de la séquence peptidique SEQ ID n°1 obtenu par la mutation, la substitution, la délétion, l'addition ou la modification d'un résidu.

Un mode de réalisation tout particulier de l'invention est représenté par une séquence nucléotidique comprenant la séquence nucléotidique SEQ ID n°1 ou son brin complémentaire.

L'invention concerne également une séquence nucléotidique codant pour un polypeptide ayant une activité de récepteur opioïde kappa humain caractérisée en ce qu'elle est choisie parmi les séquences dérivées de la SEQ ID n°1 en raison de la dégénérescence du code génétique.

Les différentes séquences nucléotidiques de l'invention peuvent être d'origine artificielle ou non. Il peut s'agir de séquences génomiques, d'ADNc, d'ARN, de séquences hybrides ou de séquences synthétiques ou semi-synthétiques. Ces séquences peuvent être obtenues par exemple par criblage de banques d'ADN (banque d'ADNc, banque d'ADN génomique) au moyen de sondes élaborées sur la base de la séquence SEQ ID n°1. De telles banques peuvent être préparées à partir de cellules de différentes origines par des techniques classiques de biologie moléculaire commes de l'homme du métier. Les séquences nucléotidiques de l'invention peuvent également être préparées par synthèse chimique, notamment selon la méthode des phosphoramidites, ou encore par des méthodes mixtes incluant la modification chimique ou enzymatique de séquences obtenues par criblage de banques.

Les séquences nucléotidiques de l'invention peuvent être utilisées pour la production des polypeptides opioïdes kappa. Le terme polypeptide opioïde kappa désigne tout polypeptide ayant une activité de récepteur opioïde kappa, et tout fragment ou dérivé d'un tel polypeptide. Pour la production de polypeptide opioïdes kappa, la partie codant pour ledit polypeptide est généralement placée sous le contrôle de signaux permettant son expression dans un hôte cellulaire. Le choix de ces signaux (promoteurs, terminateurs, etc) peut varier en fonction de l'hôte cellulaire utilisé. A cet effet, les séquences nucléotidiques de l'invention peuvent faire partie d'un vecteur, qui peut être à réplication autonome ou intégratif. Plus particulièrement, des vecteurs à réplication autonome peuvent être préparés en utilisant des séquences à réplication autonome chez l'hôte choisi. S'agissant des vecteurs intégratifs, ceux-ci peuvent être préparés par exemple en utilisant des séquences homologues à certaines régions du génome de l'hôte, permettant, par recombinaison homologue, l'intégration du vecteur. Les hôtes cellulaires utilisables pour la production des polypeptides opioïdes de l'invention par voie recombinante sont aussi bien des hôtes eucaryotes que procaryotes. Parmi les hôtes eucaryotes qui conviennent, on peut citer les cellules animales, les levures, ou les champignons. En particulier, s'agissant de levures, on peut citer les levures du genre *Saccharomyces, Kluyveromyces, Pichia, Schwanniomyces,* ou *Hansenula.* S'agissant de cellules animales, on peut citer les cellules COS, CHO, Cl27, NIH-3T3, etc. Parmi les champignons, on peut citer plus particulièrement *Aspergillus* ssp. ou *Trichoderma* ssp. Comme hôtes procaryotes, on préfère utiliser les bactéries suivantes *E. coli*, *Bacillus,* ou *Streptomyces.*

Les séquences nucléotidiques de la présente invention sont également utilisables dans le domaine pharmaceutique, soit pour la réalisation de séquences antisens utilisables dans le cadre d'une thérapie génique, soit encore pour la réalisation de sondes permettant la détection, par des expériences d'hybridation, de l'expression de récepteurs opioïdes dans des échantillons biologiques et la mise en évidence d'anomalies génétiques (polymorphisme, mutations) ou d'expressions aberrantes.

L'inhibition de l'expression de certains gènes par des séquences antisens s'est avérée être une stratégie prometteuse dans le contrôle de l'activité d'un gène. Les séquences antisens sont des séquences dont le produit de transcription est complémentaire du brin codant d'un gène donné, et est de ce fait capable d'hybrider spécifiquement avec l'ARNm transcrit, inhibant sa traduction en protéine (EP140 308). L'invention a ainsi pour objet les séquences antisens capables d'inhiber au moins partiellement la production de polypeptides opioïdes kappa tels que définis précédemment. De telles séquences peuvent être constituées par les séquences nucléotidiques définies ci-avant. Il s'agit généralement de séquences complémentaires de séquences codant pour des peptides de l'invention. De telles séquences peuvent être obtenues à partir de la séquence SEQ ID n°1, par fragmentation, etc, ou par synthèse chimique.

Comme indiqué ci-dessus, l'invention permet également la réalisation de sondes nucléotidiques, synthétiques ou non, capables de s'hydrider avec les séquences nucléotidiques définies ci-avant qui codent pour des polypeptides opioïdes de l'invention, ou avec les ARNm correspondants. De telles sondes peuvent être utilisées *in vitro* comme outil de diagnostic, pour la détection de l'expression d'un récepteur opioïde kappa, ou encore pour la mise en évidence d'anomalies génétiques (mauvais épissage, polymorphisme, mutations ponctuelles, etc). Compte tenu des activités multiples des ligands endogènes des récepteurs opioïdes, les sondes de l'invention peuvent ainsi permettre d'identifier des affections neurologique, cardiovasculaire ou psychiatrique. Ces sondes peuvent également être utilisées pour la mise en évidence et l'isolement de séquences d'acides nucléiques homologues codant pour des polypeptides opioïdes tels que définis précédemment, à partir d'autres sources cellulaires et préférentiellement de cellules d'origines humaines. Les sondes de l'invention comportent l'intégralité de la séquence SEQ ID n°1 ou de son brin complémentaire. Préférentiellement, ces sondes sont, préalablement à leur utilisation, marquées. Pour cela, différentes techniques connues de l'homme du métier peuvent être employées (marquage radioactif, enzymatique, etc). Les conditions d'hybridation dans lesquelles ces sondes peuvent être utilisées sont indiquées dans les techniques générales de clonage ci-après ainsi que dans les exemples.

L'invention a également pour objet tout polypeptide résultant de l'expression d'une séquence nucléotidique telle que définie précédemment. Il s'agit d'un polypeptide comprenant la séquence peptidique SEQ ID n° 1 ou d'un dérivé de celle-ci.

Au sens de la présente invention, la terme dérivé désigne toute molécule obtenue par une modification de nature génétique et/ou chimique de la séquence peptidique SEQ ID n° 1 et conservant une activité de récepteur opioïde kappa, par modification de nature génétique et/ou chimique, on peut entendre toute mutation, substitution, délétion, addition ou modification d'un résidu. De tels dérivés peuvent être générés dans des buts différents, tels que notamment celui d'augmenter l'affinité du peptide pour son (ses) ligand(s), celui d'améliorer ses niveaux de production, celui d'augmenter sa résistance à des protéases, celui d'augmenter et/ou de modifier son activité, ou celui de lui conférer de nouvelles propriétés pharmacocinétiques et/ou biologiques.

Préférentiellement, les polypeptides de l'invention sont des polypeptides possédant la capacité de lier la dynorphine et les dérivés du gène de la prodynorphine. Encore plus préférentiellement, ils possédent la capacité de lier les agonistes de la dynorphine tels que le U-50,488, l'éthylcétocyclasoncine et la brémozacine, et les antagonistes de la dynorphine tel que la norbinaltorphimine. Toujours selon un mode préféré, les polypeptides de l'invention sont susceptibles d'être reconnus par des anticorps reconnaissant la séquence peptidique SEQ ID n° 1 complète. De tels anticorps peuvent être générés par toute technique connue de l'homme du métier, en utilisant comme antigènes les polypeptides décrits dans la présente demande.

Comme indiqué dans les exemples, ces polypeptides peuvent être exprimés dans différents types cellulaires pour former des récepteurs opioïdes fonctionnels.

Les polypeptides de l'invention peuvent être obtenus par expression dans un hôte cellulaire d'une séquence nucléotidique telle que décrite ci-dessus, par synthèse chimique, sur la base de la séquence SEQ ID n° 1 en utilisant les techniques connues de l'homme du métier, ou par une combinaison de ces techniques.

Un autre objet de l'invention concerne les cellules recombinées capables d'exprimer à leur surface un polypeptide ayant une activité de récepteur opioïde kappa humain. Ces cellules peuvent être obtenus par introduction d'une séquence nucléotidique telle que définie ci-dessus, puis culture desdites cellules dans des conditions d'expression de ladite séquence.

Les cellules recombinées selon l'invention peuvent être aussi bien des cellules eucaryotes que procaryotes. Parmi les cellules eucaryotes qui conviennent, on peut citer les cellules animales, les levures, ou les champignons. En particulier, s'agissant de levures, on peut citer les levures du genre *Saccharomyces, Kluyveromyces, Pichia, Schwanniomyces,* ou *Hansenula.* S'agissant de cellules animales, on peut citer les cellules COS, CHO, C127, NIH-3T3, etc. Parmi les champignons, on peut citer plus particulièrement *Aspergillus ssp.* ou *Trichoderma* ssp. Comme cellules procaryotes, on préfère utiliser les bactéries suivantes *E. coli, Bacillus*, ou *Streptomyces*. Les cellules ainsi obtenues peuvent être utilisées pour mesurer la capacité de différentes molécules à se comporter comme ligand ou comme modulateur de l'activité des récepteurs opioïdes. Plus particulièrement, elles peuvent ainsi être utilisées dans un procédé de mise en évidence et d'isolement de ligands ou de modulateur de l'activité des récepteurs opioïdes, et, plus préférentiellement, d'agonistes et d'antagonistes.

Un autre objet de l'invention concerne donc un procédé de mise en évidence et/ou d'isolement de ligands des récepteurs opioïdes, selon lequel on réalise les étapes suivantes :
- on met en contact une molécule ou un mélange contenant différentes molécules, éventuellement non-identifiées, avec une cellule recombinée telle que décrite ci-dessus exprimant à sa surface un polypeptide ayant une activité de récepteur opioïde dans des conditions permettant l'interaction entre ledit polypeptide et ladite molécule dans le cas où celle-ci possèderait une affinité pour ledit polypeptide, et,
- on détecte et/ou isole les molécules liées au dit polypeptide.

Dans un mode particulier, ce procédé de l'invention est adapté à la mise en évidence et/ou l'isolement d'agonistes et d'antagonistes de la dynorphine ou autres ligands des récepteurs kappa pour les récepteurs opioïdes kappa.

Un autre objet de l'invention concerne un procédé de mise en évidence et/ou d'isolement de modulateurs des récepteurs opioïdes, selon lequel on réalise les étapes suivantes :
- on met en contact une molécule ou un mélange contenant différentes molécules, éventuellement non-identifiées, avec une cellule recombinée telle que décrite ci-dessus exprimant à sa surface un polypeptide ayant une activité de récepteur opioïde, en présence du ligand endogène dudit récepteur, dans des conditions permettant l'interaction entre ledit polypeptide et son ligand, et,
- on détecte et/ou isole les molécules capables de moduler l'activité du ligand sur ledit polypeptide.

Dans un mode particulier, ce procédé de l'invention est adapté à la mise en évidence et/ou l'isolement de modulateurs de l'activité de la dynorphine ou autres ligands des récepteurs kappa sur les récepteurs opioïdes kappa.

Un autre objet de l'invention concerne l'utilisation d'un ligand ou d'un modulateur identifié et/ou obtenu selon le procédé décrit ci-avant comme médicament. De tels ligands ou modulateurs peuvent en effet permettre de traiter certaines affections liées aux récepteurs opioïdes. En particulier, les récepteurs opioïdes kappa étant les médiateurs d'un effet analgésique, les agonistes de ces récepteurs peuvent être utilisés pour diminuer les sensations de douleur.

L'invention concerne également tout médicament comprenant comme principe actif au moins une molécule agissant sur un récepteur de l'invention. Préférentiellement la molécule est un ligand ou un modulateur identifié et/ou isolé selon le procédé décrit précédemment.

D'autres avantages de la présente invention apparaîtront à la lecture des exemples qui suivent, qui doivent être considérés comme illustratifs et non limitatifs.

### Légende des figures

Figure 1 : Organization partielle du gène du récepteur opioïde kappa humain (hKOR). Les exons sont indiqués par des boïtes et les introns par des lignes. La position des domaines transmembranaires putatifs du récepteur kappa sont indiqués par des chiffres romains. Les extrémités connues des exons sont numérotées en chiffres arabes, par rapport à leur position dans l'ADNc (fig. 2). L'exon en 3' a été séquencé jusqu'au codon terminateur TGA.
Figure 2 **:** Stratégie de clonage de l'ADNc codant pour le récepteur opioïde kappa humain.

### Techniques générales de clonage

Les méthodes classiquement utilisées en biologie moléculaire telles que les extractions préparatives d'ADN plasmidique, la centrifugation d'ADN plasmidique en gradient de chlorure de césium, l'électrophorèse sur gels d'agarose ou d'acrylamide, la purification de fragments d'ADN par électroélution, les extractions de protéines au phénol ou au phénol-chloroforme, la précipitation d'ADN en milieu salin par de l'éthanol ou de l'isopropanol, la transformation dans *Escherichia coli,* etc, sont bien connues de l'homme de métier et sont abondament décrites dans la littérature [Maniatis T. et al., "Molecular Cloning, a Laboratory Manual", Cold Spring Harbor Laboratory, Cold Spring Harbor, N.Y., 1982; Ausubel F.M. et al. (eds), "Current Protocols in Molecular Biology", John Wiley & Sons, New York, 1987].

Pour les ligatures, les fragments d' ADN sont séparés selon leur taille par électrophorèse en gels d'agarose ou d'acrylamide, extraits au phénol ou par un mélange phénol/chloroforme, précipités à l'éthanol puis incubés en présence de l'ADN ligase du phage T4 selon les recommandations du fournisseur.

Le remplissage des extrémités 5' proéminentes est effectué par le fragment de Klenow de l'ADN Polymérase I d'*E.coli* selon les spécifications du fournisseur. La destruction des extrémités 3' proéminentes est effectuée en présence de l'ADN Polymérase du phage T4 utilisée selon les recommandations du fabricant. La destruction des extrémités 5' proéminentes est effectuée par un traitement ménagé par la nucléase S1.

La mutagénèse dirigée *in vitro* par oligodéoxynucléotides synthétiques est effectuée selon la méthode développée par Taylor et al. [Nucleic Acids Res. 13 (1985) 8749-8764].

L'amplification enzymatique de fragments d'ADN par la technique dite de PCR [Polymérase-catalyzed Chain Réaction, Saiki R.K. et al., Science 230 (1985) 1350-1354 ; Mullis K.B. et Faloona F.A., Meth. Enzym. 155 (1987) 335-350].

La vérification des séquences nucléotidiques est effectuée par la méthode développée par Sanger et al. [Proc. Natl. Acad. Sci. USA, 74 (1977) 5463-5467].

Pour les expériences d'hybridation, les conditions de stringence sont basées sur Maniatis T. et al., précitée.

### 1. Clonage génomique et organization partielle du gène

Une banque génomique humaine a été criblée à l'aide d'une sonde provenant d'un ADNc codant pour un récepteur delta de souris (Kieffer et al., PNAS 1992, vol 89, 12048). La sonde est un fragment Pst I-Not I (976bp) de l'ADNc qui correspond à la majorité de la portion codante de l'ADNc. La banque a été étalée et transférée sur filtres de nitrocellulose. Ceux-ci ont été hybridés à la sonde marquée au ³²P, dans des conditions relativement stringentes (5XSSC, 5XDenhardt's, SDS O.1%, Formamide 40%, NaPPi 0.05% et ADN de sperme de saumon 100 µg/ml). Les lavages ont été réalisés en 0.1% SSC jusqu'à 50°C et les filtres exposés à un film Rayons-X pendant la nuit.

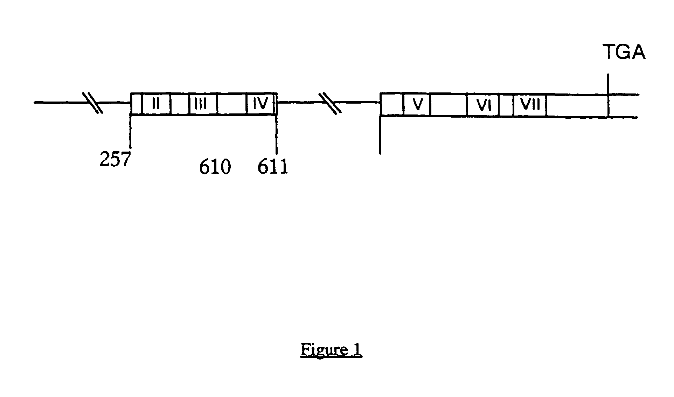

Deux clones génomiques hybridant faiblement avec la sonde ont été isolés. L'analyse des inserts par séquençage partiel indique qu'ils contiennent des régions très homologues au récepteur kappa de souris. Un exon a été identifié sur chacun des clones: l'un code pour une région correspondant topologiquement au début de la première boucle intracellulaire jusqu'à la fin du quatrième domaine transmembranaire et l'autre code pour le reste de l'extrémité C-terminale du récepteur. La topologie de cette portion du gène est représentée à la figure 1 et la position exacte des jonctions intron-exon y est indiquée.

### 2. Clonage d'un ADNc complet codant pour le récepteur kappa humain

La plupart des méthodes utilisées ("standard"), sauf précisé dans le texte, dérivent de Sambrook, J.et al., 1989 Molecular Cloning : A Laboratory Manual (Cold Spring Harbor Lab., Cold Spring Harbor, NY), 2nd Ed.

Le placenta humain exprime le récepteur kappa (Meunier et al., 1988 Life Sci., vol 43, 559). Nous avons préparé de l'ARN total de placenta humain (Chomczynski, P. et al., 1987, Anal. Biochem., vol 162, 156) et avons synthétisé de l'ADNc à partie de cet ARN, à l'aide d'une amorce nucléotidique au hasard ("random hexamer", Pharmacia) et de Moloney Murine Leukemia Virus réverse transcriptase (BRL), dans des conditions standard.

Nous avons ensuite amplifié l'ADNc codant pour le récepteur kappa humain par PCR (Polymerase Chain reaction) en utilisant des portions de la séquence humaine obtenue à partir de l'analyse des clones génomiques pour la conception d'amorces spécifiques. L'extrémité 5' de la partie codante du gène nous manquant, nous avons utilisé un oligonucléotide dérivé à partir d'une séquence kappa de souris pour l'amplification à partir de l'extrémité 5'.

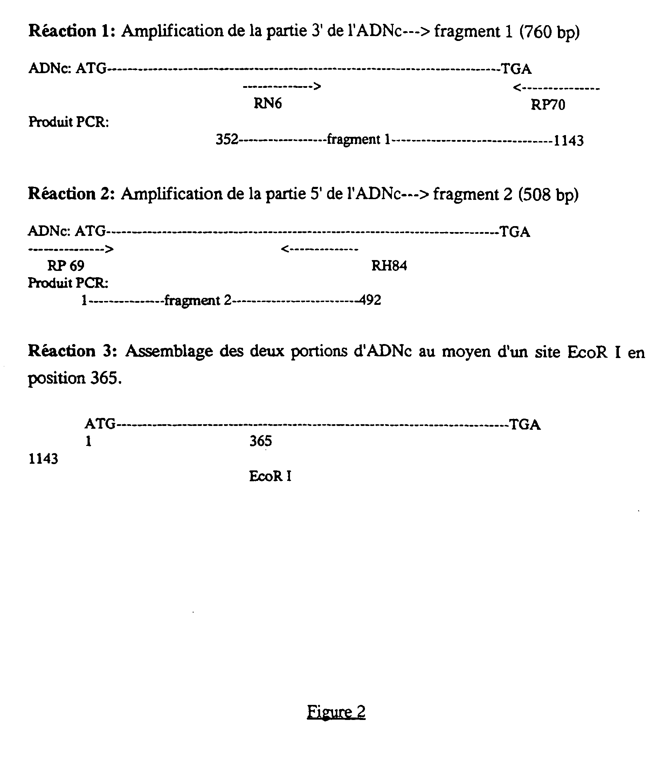

L'utilisation de deux amorces situées aux extrémités 5' (souris) et 3' (humain) de la région codante s'étant avérée peu efficace pour la production d'un ADNc complet, nous avons procédé en trois étapes et la stratégie utilisée est présentée sur la figure 2 (les chiffres indiquent la position précise des nucléotides dans la séquence complète de l'ADNc représentée sur la séquence SEQ ID n° 1).

La séquence des oligonucléotides utilisés est la suivante :
RP69: 5' GAGAGCTCGCGGCCGCGAGCTGCAGCGCTCACCATG (SEQ ID 2)
RH84: 5' CACGGGGTGGCACACGGCAA (SEQ ID n° 3)
RN6: 5' GTCTACTTGATGAATTCCTGG (SEQ ID n° 4)
RP70: 5' AGACCCAAGCTTGCCCGGGTCCACGACTAGTCATACTGG (SEQ ID n° 5)

Les séquences hybridant à l'ADNc sont indiquées en gras, les autres nucléotides étant présent pour faciliter l'étape ultérieure de clonage des fragments produits par la réaction de PCR.
Les oligonucléotides RP70, RH84 et RN6 sont dérivés de la séquence kappa humaine. L'oligonucléotide RP69 correspond à une séquence 5' non codante de souris (incluant l'ATG initiateur à l'extrémité 3') afin de ne pas introduire de séquence murine dans l'ADNc humain. La position précise des régions de l'ADNc qui hybrident aux 4 amorçes est indiquée à la fig. 2.

Les différentes réactions d'amplification ont été réalisées dans les conditions suivantes :
Réaction 1: Amorces RN6 et RP70. La réaction est réalisée en présence de Taq Polymérase (Cetus) dans des conditions de PCR standard, excepté l'addition de DMSO 5% dans le milieu d'incubation. L'amplification se fait durant 40 cycles (1min à 94°C, 1 min à 55°C, 1 min à 72°C). La dernière étape d'élongation se réalise pendant 10 min à 72°C. On obtient un fragment d'ADN de la taille attendue (760 bp)= fragment 1.
Réaction 2: Les conditions d'amplification sont les mêmes que pour la réaction 1. Deux amplifications successives ont été nécessaires: la première utilise les amorces RP69 et RP70 et aboutit à l'obtention d'un mélange de fragments d'ADN de taille comprise entre 1 et 1.3 kb, contenant l'ADNc complet. Ce mélange est purifié après électrophorèse à partir d'un gel d'agarose 1% par le procédé GeneClean et réamplifié à l'aide des oligonucléotides RP69 et RH84. On obtient alors un fragment d'ADN de la taille attendue (508 bp)=fragment 2.

### Clonage de l'ADNc complet:

Les fragments PCR sont réparés et clonés dans les extrémités franches du vecteur pBluescript (Stratagene) linéarisé par EcoR V. Les inserts sont séquencés dans les deux sens sur un séquenceur d'ADN automatique (373A DNA, Applied Bioystems Inc.) .Puis les deux inserts sont excisés du plasmide en présence de EcoR I (coupe côté 3' du fragment 2 et côté 5' du fragment 1) et d'un enzyme de restriction du polylinker. Les fragments sont purifiés à partir d'un gel d'agarose 1% par le procédé gene Clean et co- ligués dans le vecteur pcDNA/Amp (Invitrogen) pour l'expression transitoire du récepteur en cellules Cos.

Les deux réactions d'amplification ont produit les fragments 1 et 2 attendus. Dans le cas du fragment 2, l'utilisation d'un oligonucléotide dérivé d'une séquence de souris à l'extrémité 5' a permis l'amplification de l'ADNc humain.

Les deux fragments se recouvrent sur 140 bp. La séquence de recouvrement est identique dans les deux fragments. Les séquences des deux fragments sont comparées à la séquence déterminée à partir de l'ADN génomique (position 257 à position 1143) et trouvée parfaitement identique. Concernant la portion de séquence 1 à 256, dont la séquence génomique nous est inconnue, nous avons vérifié que l'amplification par PCR n'a pas introduit d'erreurs: nous avons comparé la séquence du fragment 2 obtenu à partir de trois réactions d'amplification différentes et l'avons trouvée identique dans les 3 cas.

La séquence de l'ADNc codant pour le récepteur kappa humain selon l'invention, ainsi que la séquence protéique déduite, sont présentées sur la séquence SEQ ID n° 1.

### 3. Conclusion: structure primaire du récepteur kappa humain

Nous avons utilisé une sonde codant pour un récepteur opioïde delta de souris afin de cribler une banque génomique humaine à moyenne stringence et d'isoler des fragments d'ADN génomique homologues.

Nous avons identifié deux exons, qui au vu d'une comparaison de séquence, codent vraisemblablement pour le récepteur opioïde kappa humain. Nous avons isolé un ADNc dérivé de ce gène (partie codante) à partir d'un tissu humain (placenta) et avons analysé sa structure primaire (SEQ ID n° 1).

L'ADNc a une taille de 1101 paires de bases et code pour une protéine de 380 acides aminés . La séquence nucléotidique est homologue à 86,7% à l'ADNc de souris codant pour un récepteur opioïde kappa. Les zones de divergence les plus importantes sont situées dans lés régions N- et C-terminales. Ces mêmes zones sont également les régions les moins conservées entre les trois sous-types mu, delta et kappa.

### 4. Etude pharmacologique du récepteur K56

Le vecteur pcDNA/Amp contenant le cDNA codant pour le récepteur opioïde selon l'invention isolé dans l'exemple 2 est utilisé pour transfecter des cellules Cos-1. Les membranes des cellules transfectées obtenues ont ensuite été préparées et testées pour leur capacité à lier certains ligands opioïdes marqués.

Le plasmide vecteur pcDNA/Amp purifié sur chlorure de césium est utilisé pour transfecter les cellules Cos-1 en utilisant la technique au DEAE-dextran.

72 heures après la transfection, les cellules recombinantes sont récoltées et les membranes sont préparées de la manière suivante : les culots cellulaires sont repris, à 4°C, dans 60 ml de tampon Tris-HCl 50 mM pH 7,4; EDTA 10 mM, homogénéisés et centrifugés à 1100 g pendant 10 minutes. Le culot est ensuite repris une seconde fois dans 30 ml du même tampon, homogénéisé et centrifugé. Les 2 surnageants sont réunis et centrifugés à 110 000 g pendant 15 minutes. Le culot membranaire est alors repris dans 5ml du même tampon, aliquoté et conservé à - 80 °C. Des expériences de liaison à saturation et de compétition sont ensuite réalisées sur ces membranes en présence de différents ligands. Pour cela, les échantillons de membrane (15-30 µg de protéines) sont incubés 2 heures à 25°C en présence de ³H-diprénorphine ou ³H-U-69,593, avec ou sans compétiteur, dans un volume final de 1 ml de tampon Tris-HCl 50 mM (pH 7,4); EDTA 10 mM. La réaction est ensuite stopée par filtration sous vide sur filtres Whatman GF/B, et rinçage 3 fois avec 3 ml du tampon froid. Les valeurs de Ki sont obtenues en suivant l'équation de Cheng et Prussof : Ki = IC50 / (1 + L/Kd). La radioactivité a été mesurée avec un compteur β.

## Revendications

1. Séquence nucléotidique codant pour un polypeptide ayant une activité de récepteur opioïde kappa humain, le dit polypeptide comprenant
a. la séquence peptidique SEQ ID n°1, ou
b. un dérivé de la séquence peptidique SEQ ID n°1 obtenu par la mutation, la substitution, la délétion, l'addition ou la modification d'un résidu.

2. Séquence nucléotidique comprenant
a. la séquence SEQ ID n°1, ou
b. la séquence complémentaire de la séquence (a).

3. Séquence nucléotidique codant pour un polypeptide ayant une activité de récepteur opioïde kappa humain **caractérisée en ce qu'**elle est choisie parmi les séquences dérivées de la SEQ ID n°1 en raison de la dégénérescence du code génétique.

4. Séquence nucléotidique selon la revendication 1, 2 ou 3 **caractérisée en ce qu'**elle est choisie parmi les séquences d'ADN génomique, d'ADNc, d'ARN, les séquences hybrides ou les séquences synthétiques ou semi-synthétiques.

5. Séquence selon l'une des revendications 1 à 4 **caractérisée en ce que** la partie codant pour ledit polypeptide est placée sous le contrôle de signaux permettant son expression dans un hôte cellulaire.

6. Polypeptide comprenant la séquence peptidique SEQ 10 n°1 ou un dérivé de cette séquence obtenu par la mutation, la substitution, la délétion, l'addition ou la modification d'un résidu, ledit polypeptide ayant une activité de récepteur opioïde kappa.

7. Séquence antisens capable d'inhiber au moins partiellement la production d'un polypeptide selon la revendication 6.

8. Utilisation d'une sonde ayant une séquence selon la revendication 1, 2 ou 3 pour la détection de l'expression d'un récepteur opioïde ; ou pour la mise en évidence d'anomalies génétiques (mauvais épissage, polymorphisme, mutations ponctuelles, etc) ; ou pour identifier des affections neurologique, cardiovasculaire ou psychiatrique comme étant liées aux récepteurs opioïdes ; ou encore pour la mise en évidence et l'isolement de séquences d'acides nucléiques homologues codant pour des polypeptides opioïdes.

9. Cellule recombinée capable d'exprimer à sa surface un polypeptide selon la revendication 6. ladite cellule étant choisie parmi les cellules procaryotes, les levures, les champignons et les cellules COS, CHO, C127 et NIH-3T3.

10. Procédé de mise en évidence et/ou d'isolement de ligands des récepteurs opioïdes, **caractérisé en ce que** l'on réalise les étapes suivantes :
- on met en contact une molécule ou un mélange contenant différentes molécules. éventuellement non-identifiées, avec une cellule recombinée selon la revendication 9, exprimant à sa surface un polypeptide opioïde, dans des conditions permettant l'interaction entre ledit polypeptide et ladite molécule dans le cas où celle-ci posséderait une affinité pour ledit polypeptide, et ,
- on détecte et/ou isole les molécules liées au dit polypeptide.

11. Procédé selon la revendication 10 pour la mise en évidence et/ou l'isolement de ligands des récepteurs opioïdes kappa.

12. Procédé de mise en évidence et/ou d'isolement de modulateurs des récepteurs opioïdes, **caractérisé en ce que** l'on réalise les étapes suivantes :
- on met en contact une molécule ou un mélange contenant différentes molécules, éventuellement non-identifiées, avec une cellule recombinée selon la revendication 9 exprimant à sa surface un polypeptide ayant une activité de récepteur opioïde, en présence d'un ligand dudit récepteur, dans des conditions permettant l'interaction entre ledit polypeptide et le ligand, et,
- on détecte et/ou isole les molécules capables de moduler l'activité du ligand sur ledit polypeptide.

## Claims

1. A nucleotide sequence coding for a polypeptide having a human kappa opioid receptor activity, said polynucleotide comprising:
a. the peptide sequence SEQ ID NO:1; or
b. a derivative of the peptide sequence SEQ ID NO: 1 obtained by mutation, substitution, deletion, addition or modification of one residue.

2. A nucleotide sequence comprising:
a. the sequence SEQ ID NO: 1; or
b. the complementary sequence of sequence (a).

3. A nucleotide sequence coding for a polypeptide having a human kappa opioid receptor activity, **characterized in that** it is selected from sequences derived from SEQ ID NO: 1 due to degeneracy of the genetic code.

4. A nucleotide sequence according to claim 1, 2 or 3, **characterized in that** it is selected from sequences of genomic DNA, cDNA, RNA, hybrid sequences or synthetic or semi-synthetic sequences.

5. A sequence according to one of claims 1 to 4, **characterized in that** the portion coding for said polypeptide is placed under the control of signals allowing its expression in a host cell.

6. A polypeptide comprising peptide sequence SEQ ID NO: 1 or a derivative of said sequence obtained by mutation, substitution, deletion, addition or modification of one residue, said polypeptide having a kappa opioid receptor activity.

7. An anti-sense sequence capable of at least partially inhibiting the production of a polypeptide according to claim 6.

8. Use of a probe having a sequence according to claim 1, 2 or 3 to detect expression of an opioid receptor; or to demonstrate genetic anomalies (poor splicing, polymorphism, point mutations, etc); or to identify neurological, cardiovascular or psychiatric disorders as being linked to opioid receptors; or to demonstrate and isolate homologous nucleic acid sequences coding for opioid polypeptides.

9. A recombinant cell capable of expressing a polypeptide according to claim 6 on its surface, said cell being selected from prokaryotic cells, yeasts, fungi and COS, CHO, C127 and NIH-3T3 cells.

10. A method for demonstrating and/or isolating ligands of opioid receptors, **characterized in that** the following steps are carried out:
• bringing a molecule or a mixture containing different molecules, possibly not identified, into contact with a recombinant cell according to claim 9 expressing an opioid polypeptide on its surface, under conditions allowing interaction between said polypeptide and said molecule in the case in which it might have an affinity for said polypeptide; and
• detecting and/or isolating molecules bound to said polypeptide.

11. A method according to claim 10, to demonstrate and/or isolate ligands of kappa opioid receptors.

12. A method for demonstrating and/or isolating modulators for opioid receptors, **characterized in that** the following steps are carried out:
• bringing a molecule or a mixture containing different molecules, possibly not identified, into contact with a recombinant cell according to claim 9 expressing a polypeptide having an opioid receptor activity on its surface into the presence of a ligand for said receptor, under conditions allowing interaction between said polypeptide and said ligand; and
• detecting and/or isolating molecules capable of modulating the activity of said polypeptide.

## Patentansprüche

1. Nukleotidsequenz die für ein Polypeptid codiert, das eine Aktivität als menschlicher kappa-Opioidrezeptor besitzt, wobei das Polypeptid umfasst
a) die Peptidsequenz SEQ ID Nr.1, oder
b) ein Derivat der Peptidsequenz SEQ ID Nr.1, das durch Mutation, Substitution, Deletion, Addition oder Modifikation eines Restes erhalten wird.

2. Nukleotidsequenz umfassend
a) die Sequenz SEQ ID Nr.1, oder
b) zu der Sequenz (a) komplementäre Sequenz.

3. Nukleotidsequenz die für ein Polypeptid codiert, das eine Aktivität als menschlicher kappa-Opioidrezeptor besitzt, **dadurch gekennzeichnet, dass** sie ausgewählt ist aus den von der SEQ ID Nr.1 aufgrund der Degeneration des genetischen Codes abgeleiteten Sequenzen.

4. Nukleotidsequenz gemäß Anspruch 1, 2 oder 3, **dadurch gekennzeichnet, dass** sie ausgewählt ist aus genomischen DNA-, cDNA-, RNA-Sequenzen, Hybridsequenzen oder synthetischen oder semi-synthetischen Sequenzen.

5. Sequenz gemäß einem der Ansprüche 1 bis 4, **dadurch gekennzeichnet, dass** der Teil, der für das Polypeptid codiert, sich unter der Kontrolle von Signalen befindet, die dessen Expression in einer Wirtszelle erlauben.

6. Polypeptid umfassend die Peptidsequenz SEQ ID Nr.1 oder ein Derivat dieser Sequenz, die durch Mutation, Substitution, Deletion, Addition oder Modifikation eines Restes erhalten wird, wobei das Polypeptid eine Aktivität als kappa-Opioidrezeptor besitzt.

7. Antisense-Sequenz die in der Lage ist, mindestens teilweise die Erzeugung eines Polypeptids gemäß Anspruch 6 zu inhibieren.

8. Verwendung einer Sonde, die eine Sequenz gemäß Anspruch 1, 2 oder 3 besitzt, zur Detektion der Expression eines Opioidrezeptors; oder zur Aufdeckung genetischer Anomalien (Fehlspleißen, Polymorphismus, Punktmutationen, usw.); oder zur Identifikation neurologischer, kardiovaskulärer oder psychiatrischer Leiden, die mit Opioidrezeptoren in Verbindung gebracht werden; oder auch zur Aufdeckung und Isolierung von homologen Nukleinsäuresequenzen, die für Opioid-Polypeptide codieren.

9. Rekombinante Zelle die in der Lage ist, auf ihrer Oberfläche ein Polypeptid gemäß Anspruch 6 zu exprimieren, wobei die Zelle ausgewählt ist aus prokaryotischen Zellen, Hefen, Pilzen und COS-, CHO-, C127- und NIH-3T3-Zellen.

10. Verfahren zur Aufdeckung und/oder Isolierung von Liganden von Opioidrezeptoren, **dadurch gekennzeichnet, dass** folgenden Schritte ausgeführt werden:
- Inkontaktbringen eines Moleküls oder einer Mischung, die verschiedene Moleküle enthält, möglicherweise unbestimmter Art, mit einer rekombinanten Zelle gemäß Anspruch 9, die auf ihrer Oberfläche ein Opioid-Polypeptid exprimiert, unter Bedingungen, die die Interaktion zwischen dem Polypeptid und dem Molekül für den Fall erlauben, dass dieses eine Affinität für das Polypeptid besitzt, und
- Nachweis und/oder Isolierung der mit diesem Polypeptid verbundenen Moleküle.

11. Verfahren gemäß Anspruch 10 zur Aufdeckung und/oder Isolierung von Liganden von kappa-Opioidrezeptoren.

12. Verfahren zur Aufdeckung und/oder Isolierung von Modulatoren von Opioidrezeptoren, **dadurch gekennzeichnet, dass** die folgenden Schritte ausgeführt werden:
- Inkontaktbringen eines Moleküls oder einer Mischung, die verschiedene Moleküle enthält, möglicherweise unbestimmter Art, mit einer rekombinanten Zelle gemäß Anspruch 9, die auf ihrer Oberfläche ein Polypeptid exprimiert, das eine Aktivität als Opioidrezeptor besitzt, in Gegenwart eines Liganden dieses Rezeptors unter Bedingungen, die die Interaktion zwischen dem Polypeptid und dem Liganden erlauben, und
- Nachweis und/oder Isolierung der Moleküle, die in der Lage sind, die Aktivität des Liganden auf dem Polypeptid zu modulieren.


### LISTE DE SEQUENCES

(1) INFORMATION GENERALE:
   (i) DEPOSANT:
      (A) NOM: Universite Louis Pasteur
      (B) RUE: 11 rue Humann
      (C) VILLE: STRASBOURG
      (E) PAYS: FRANCE
      (F) CODE POSTAL: 67085
   (ii) TITRE DE L' INVENTION: RECEPTEUR OPIOIDE KAPPA HUMAIN, ACIDES NUCLEIQUES ET UTILISATIONS
   (iii) NOMBRE DE SEQUENCES: 5
   (iv) FORME LISIBLE PAR ORDINATEUR:
      (A) TYPE DE SUPPORT: Tape
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 1143 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (ix) CARACTERISTIQUE ADDITIONELLE:
      (A) NOM/CLE: CDS
      (B) EMPLACEMENT: 1..1143
   (xi) DESCRIPTION DE LA SEQUENCE: SEQ ID NO: 1: 
ATG GAC TCC CCG ATC CAG ATC TTC CGC GGG GAG CCG GGC CCT ACC TGC 48
Met Asp Ser Pro Ile Gln Ile Phe Arg Gly Glu Pro Gly Pro Thr Cys
1               5                   10                  15

GCC CCG AGC GCC TGC CTG CCC CCC AAC AGC AGC GCC TGG TTT CCC GGC 96
Ala Pro Ser Ala Cys Leu Pro Pro Asn Ser Ser Ala Trp Phe Pro Gly
            20                  25                  30

TGG GCC GAG CCC GAC AGC AAC GGC AGC GCC GGC TCG GAG GAC GCG CAG 144
Trp Ala Glu Pro Asp Ser Asn Gly Ser Ala Gly Ser Glu Asp Ala Gln
        35                  40                  45

CTG GAG CCC GCG CAC ATC TCC CCG GCC ATC CCG GTC ATC ATC ACG GCG 192
Leu Glu Pro Ala His Ile Ser Pro Ala Ile Pro Val Ile Ile Thr Ala
    50                  55                  60

GTC TAC TCC GTA GTG TTC GTC GTG GGC TTG GTG GGC AAC TCG CTG GTC 240
Val Tyr Ser Val Val Phe Val Val Gly Leu Val Gly Asn Ser Leu Val
65                  70                  75                  80

ATG TTC GTG ATC ATC CGA TAC ACA AAG ATG AAG ACA GCA ACC AAC ATT 288
Met Phe Val Ile Ile Arg Tyr Thr Lys Met Lys Thr Ala Thr Asn Ile
                85                  90                  95
 
TAC ATA TTT AAC CTG GCT TTG GCA GAT GCT TTA GTT ACT ACA ACC ATG 336
Tyr Ile Phe Asn Leu Ala Leu Ala Asp Ala Leu Val Thr Thr Thr Met
            100                 105                 110

CCC TTT CAG AGT ACG GTC TAC TTG ATG AAT TCC TGG CCT TTT GGG GAT 384
Pro Phe Gln Ser Thr Val Tyr Leu Met Asn Ser Trp Pro Phe Gly Asp
        115                 120                 125

GTG CTG TGC AAG ATA GTA ATT TCC ATT GAT TAC TAC AAC ATG TTC ACC 432
Val Leu Cys Lys Ile Val Ile Ser Ile Asp Tyr Tyr Asn Met Phe Thr
    130                 135                 140

AGC ATC TTC ACC TTG ACC ATG ATG AGC GTG GAC CGC TAC ATT GCC GTG 480
Ser Ile Phe Thr Leu Thr Met Met Ser Val Asp Arg Tyr Ile Ala Val
145                 150                 155                 160

TGC CAC CCC GTG AAG GCT TTG GAC TTC CGC ACA CCC TTG AAG GCA AAG 528
Cys His Pro Val Lys Ala Leu Asp Phe Arg Thr Pro Leu Lys Ala Lys
                165                 170                 175

ATC ATC AAT ATC TGC ATC TGG CTG CTG TCG TCA TCT GTT GGC ATC TCT 576
Ile Ile Asn Ile Cys Ile Trp Leu Leu Ser Ser Ser Val Gly Ile Ser
            180                 185                 190

GCA ATA GTC CTT GGA GGC ACC AAA GTC AGG GAA GAC GTC GAT GTC ATT 624
Ala Ile Val Leu Gly Gly Thr Lys Val Arg Glu Asp Val Asp Val Ile
        195                 200                 205

GAG TGC TCC TTG CAG TTC CCA GAT GAT GAC TAC TCC TGG TGG GAC CTC 672
Glu Cys Ser Leu Gln Phe Pro Asp Asp Asp Tyr Ser Trp Trp Asp Leu
    210                 215                 220

TTC ATG AAG ATC TGC GTC TTC ATC TTT GCC TTC GTG ATC CCT GTC CTC 720
Phe Met Lys Ile Cys Val Phe Ile Phe Ala Phe Val Ile Pro Val Leu
225                 230                 235                 240

ATC ATC ATC GTC TGC TAC ACC CTG ATG ATC CTG CGT CTC AAG AGC GTC 768
Ile Ile Ile Val Cys Tyr Thr Leu Met Ile Leu Arg Leu Lys Ser Val
                245                 250                 255

CGG CTC CTT TCT GGC TCC CGA GAG AAA GAT CGC AAC CTG CGT AGG ATC 816
Arg Leu Leu Ser Gly Ser Arg Glu Lys Asp Arg Asn Leu Arg Arg Ile
            260                 265                 270

ACC AGA CTG GTC CTG GTG GTG GTG GCA GTC TTC GTC GTC TGC TGG ACT 864
Thr Arg Leu Val Leu Val Val Val Ala Val Phe Val Val Cys Trp Thr
        275                 280                 285

CCC ATT CAC ATA TTC ATC CTG GTG GAG GCT CTG GGG AGC ACC TCC CAC 912
Pro Ile His Ile Phe Ile Leu Val Glu Ala Leu Gly Ser Thr Ser His
    290                 295                 300

AGC ACA GCT GCT CTC TCC AGC TAT TAC TTC TGC ATC GCC TTA GGC TAT 960
Ser Thr Ala Ala Leu Ser Ser Tyr Tyr Phe Cys Ile Ala Leu Gly Tyr
305                 310                 315                 320

ACC AAC AGT AGC CTG AAT CCC ATT CTC TAC GCC TTT CTT GAT GAA AAC 1008
Thr Asn Ser Ser Leu Asn Pro Ile Leu Tyr Ala Phe Leu Asp Glu Asn
                325                 330                 335

TTC AAG CGG TGT TTC CGG GAC TTC TGC TTT CCA CTG AAG ATG AGG ATG 1056
Phe Lys Arg Cys Phe Arg Asp Phe Cys Phe Pro Leu Lys Met Arg Met
 
            340                 345                 350

GAG CGG CAG AGC ACT AGC AGA GTC CGA AAT ACA GTT CAG GAT CCT GCT 1104
Glu Arg Gln Ser Thr Ser Arg Val Arg Asn Thr Val Gln Asp Pro Ala
        355                 360                 365

TAC CTG AGG GAC ATC GAT GGG ATG AAT AAA CCA GTA TG 1143
Tyr Leu Arg Asp Ile Asp Gly Met Asn Lys Pro Val
    370                 375                 380
(2) INFORMATION POUR LA SEQ ID NO: 2:
   (i) CARACTERISTIQUES DE LA SEQUENCE:
      (A) LONGUEUR: 36 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: NON
   (xi) DESCRIPTION DE LA SEQUENCE: SEQ ID NO: 2:
      GAGAGCTCGC GGCCGCGAGC TGCAGCGCTC ACCATG 36
(2) INFORMATION POUR LA SEQ ID NO : 3:
   (i) CARACTERISTIQUES DE LA SEQUENCE:
      (A) LONGUEUR: 20 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: NON
   (xi) DESCRIPTION DE LA SEQUENCE: SEQ ID NO: 3:
      CACGGGGTGG CACACGGCAA 20
(2) INFORMATION POUR LA SEQ ID NO: 4:
   (i) CARACTERISTIQUES DE LA SEQUENCE:
      (A) LONGUEUR: 36 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: NON
   (xi) DESCRIPTION DE LA SEQUENCE: SEQ ID NO: 4:
      GTCTACTTGA TGAATTCCTG G 21
(2) INFORMATION POUR LA SEQ ID NO: 5:
   (i) CARACTERISTIQUES DE LA SEQUENCE:
      (A) LONGUEUR: 39 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: NON
   (xi) DESCRIPTION DE LA SEQUENCE: SEQ ID NO: 5:
      AGACCCAAGC TTGCCCGGGT CCACGACTAG TCATACTGG 39